# EUROPÄISCHE PATENTANMELDUNG

(11) **EP 0 948 971 A1**
(43) Veröffentlichungstag der Anmeldung: **13.10.1999**
(21) Anmeldenummer: 98810297.6
(22) Anmeldetag: 07.04.1998
(51) Int. Cl.: A61M 39/28, F16K 7/06

(54) **Katheterventil**

(71) Anmelder: Pharmelan Medizinprodukte AG, 9493 Mauren (LI)
(72) Erfinder: Schönbächler, Peter, 9493 Mauren (LI)
(74) Vertreter: Hasler, Erich, Dr.

(57) **Zusammenfassung**

Ein Katheterventil 11 besitzt einen in einem Gehäuse (13) angeordneten, verschwenkbaren Betätigungshebel (17), mit welchem eine das Gehäuse (13) durchsetzende elastisch verformbare Leitung (15) verschliessbar ist. Der Betätigungshebel (17) ist mittels erster und zweiter Zapfen (41,43) und erster und zweiter Führungsbahnen (51,53) im Gehäuse (13) schwenkbar gelagert und in Längsrichtung verschiebbar geführt. Federn (19), welche beidseits der Leitung 15 angeordnet sind, halten den Betätigungshebel (17) unter einer gewissen Vorspannung, welche in Richtung auf die Leitung (15) wirkt. In der Schliessstellung ist der Betätigungshebel (17) schwenkbar, und die Leitung (15) wird durch den unter Federvorspannung stehenden Betätigungshebel (17) so weit zusammengedrückt, dass ein Flüssigkeitsdurchtritt unmöglich ist. In der Offenstellung ist der Betätigungshebel (17) in einer Schwenklage, in welcher der Leitungsquerschnitt im wesentlich frei ist, festgestellt. Zu diesem Zweck besitzt der Schenkel (55) der zweiten Führungsbahn (53) eine Ausbuchtung (56), welche als Rastposition für den zweiten Zapfen (43) dient. In der Offenstellung ist der Betätigungshebel (17) ebenfalls durch die Federkraft der Federn (19) gehalten. Die Federn (19) sind Elastomerstreifen, die im Gehäuse schwenkbar angeordnet sind.

## Beschreibung

Die vorliegende Erfindung betrifft ein Katheterventil mit einem Gehäuse, einer elastisch verformbaren Leitung, welche sich durch das Gehäuse erstreckt, Mitteln, um die Leitung zu verschliessen und zu öffnen; und einer ersten und einer zweiten Anschlussstelle zum Verbinden der Leitung beispielsweise mit einem Katheter und einem Auffangbehälter.

Die EP-A-0 150 666 offenbart ein Katheterventil mit einem Gehäuse und einem elastisch verformbaren Schlauch, welcher durch eine Druckrolle verschliessbar ist. Die Druckrolle ist in einer Führungsbahn verschiebbar geführt. Sie gibt in einer unwirksamen Endlage den Schlauch frei und drückt in einer wirksamen Endlage den Schlauch zusammen und schliesst denselben ab. In der wirksamen Endlage ist die Druckrolle durch den elastischen Schlauch in einer Rastposition gehalten. Der Schlauch weist zwei verdickte Endteile auf, die über eine Schulter in einen dünnwandigen Mittelteil übergehen, wobei das Gehäuse zwischen den Schultern gehalten und positioniert ist. Dieses Ventil ist einfach im Aufbau und in der Bedienung. Nachteilig hingegen ist, dass der Benützer das Ventil nur mit einem bestimmten Kraftaufwand schliessen kann und dass durch eine unbeabsichtigte Manipulation das Ventil leicht geöffnet werden kann, da der elastische Schlauch die Druckrolle in die unwirksame Endlage drückt.

Ein anderes bekanntes, selbstschliessendes Ventil hat den Nachteil, dass zum Wasserlösen das Ventil entgegen der Kraft einer Feder permanent gedrückt werden muss. Da gerade bei älteren Leuten das Wasserlösen mehr als eine Minute dauern kann, können solche Patienten das Ventil nur mit Mühe eine solch lange Zeit offen halten.

Aufgabe der vorliegenden Erfindung ist es, ein selbstschliessendes Katheterventil bereitzustellen, welches einfach in Aufbau und Bedienung ist. Das Ventil soll mit einem minimalen Kraftaufwand betätigbar sein, sodass auch ältere Leute, welche in der Motorik behindert sind, dieses gut bedienen können. Weiter soll das Ventil einhändig bedienbar sein. Auch soll das Ventil ergonomisch ausgebildet sein, damit sich bettlägerige Patienten am Ventil nicht wund scheuern.

Erfindungsgemäss wird das Ziel realisiert durch Schliessmittel mit wenigstens einem im Gehäuse in wenigstens einer bestimmten Lage verschwenkbaren Betätigungshebel, welcher wenigstens eine erste und eine zweite Stellung einnehmen kann, wobei die Leitung in der ersten Stellung offen und in der zweiten Stellung verschlossen ist, Federmittel, welche mit dem Betätigungshebel in Verbindung stehen und diesen wenigstens in der ersten oder zweiten Stellung zu halten vermögen, und Mittel, um den Betätigungshebel in wenigstens der zweiten oder der ersten Stellung festzustellen.

Dieses Ventil hat den Vorteil, dass es nur aus wenigen Einzelteilen besteht und sich daher für eine maschinelle Fertigung eignet. Die Einzelteile sind aus Kunststoff kostengünstig herstellbar. Das Ventil ist`insbesondere als Harnkatheterventil für den extra korporalen Einsatz bestimmt. Es kann grundsätzlich so konzipiert sein, dass das Schliessen des Ventils entgegen der Kraft der Federmittel erfolgt, wobei der Betätigungshebel dann in der Schliessstellung arretiert wird. Es kann beispielsweise ein separates Sperrglied, z.B. Riegel oder Schieber, vorgesehen sein, welches den Hebel in der einen oder der anderen Stellung arretiert.

Vorteilhaft sind die Federmittel so angeordnet, dass die auf den Betätigungshebel wirkende Federkraft die Leitung verschlossen hält, und die Feststellmittel den Hebel in der ersten Stellung, d.h. in der Offenstellung, arretieren. Diese Ausführungsform hat den Vorteil, dass für das Schliessen des Ventils nur die Arretierung gelöst werden muss. Das Schliessen der Leitung wird dann durch die Federkraft der Federmittel bewirkt.

In einer vorteilhaften Ausführungsform bestehen die Feststellmittel in wenigstens einer Rastposition, in welche der Betätigungshebel verschiebbar ist. Zweckmässigerweise ist die Rastposition eine in einem Winkel zur kreisförmigen Schwenkbewegung des Betätigungshebels angeordnete erste Führungsbahn, entlang welcher der Betätigungshebel verschiebbar ist. Zu diesem Zweck kann der Betätigungshebel wenigsten einen ersten Zapfen aufweisen, welcher im Gehäuse schwenkbar und in Leitungslängsrichtung verschiebbar ist. Alternativ kann der Hebel eine Rinne und das Gehäuse einen feststehenden Zapfen aufweisen, welche zusammen eine Führung für den Hebel bilden. Zwecks Bildung einer Arretierung kann der Hebel beispielsweise eine Ausnehmung oder einen Vorsprung aufweisen, welcher mit einem geeignet ausgebildeten Gehäuseteil zusammenwirken kann. Alternativ kann jedoch auch ein separates Arretierglied, z.B. ein Riegel, zur Feststellung des Hebels in der Offenstellung vorgesehen sein. Vorteilhaft weist der Betätigungshebel wenigstens einen zweiten Zapfen auf, welcher im Gehäuse in einer zweiten kurven- oder kreisförmigen Führungsbahn, z.B. einer Nut, geführt ist, welche so angeordnet ist, dass der Betätigungshebel die Leitung in der einen Stellung verschliesst und in der anderen offen hält. Mittels des wenigstens einen zweiten Zapfens und der zweiten Führungsbahn kann die Auslenkung des Betätigungshebels begrenzt werden. Zweckmässigerweise weist die zweite Führungsbahn eine Rastposition zur Aufnahme des zweiten Zapfens auf. Die Federmittel können dazu dienen, den Betätigungshebel sowohl in der Offen- wie in der Schliessstellung festzustellen.

Obwohl grundsätzlich verschiedene Arten von Federn zum Einsatz kommen können, z.B. Druckfedern, bestehen die Federmittel vorteilhaft aus wenigstens einer Blattfeder. Diese kann zwar aus Metall sein, besteht jedoch zweckmässigerweise aus einem elastomeren Kunststoff, da Kunststoffe in der Medizintechnik materialtechnisch weniger problematisch und in der Herstellung kostengünstiger sind. Vorteilhaft ist beidseitig der Leitung je ein Federelement vorgesehen. Durch zwei Federelemente kann die Leitung zuverlässig verschlossen gehalten werden.

Vorteilhaft ist die Feder eine längliche Kunststoffeder, und die Enden weisen quer zur Längsrichtung des Streifens sich erstreckende zylindrische Verdickungen auf, welche im Gehäuse schwenkbar angeordnet sind. Eine solche Feder hat den Vorteil, dass die zur Betätigung nötige Kraft über die ganze Federwegstrecke praktisch gleich bleibt - im Unterschied zu einer Druckfeder, wo die Kraft immer mehr zunimmt, je stärker die Feder zusammengedrückt wird.

Vorteilhaft ist das Ventil vollständig aus Kunststoff und die Leitung aus Silikonkunststoff, welche formschlüssig ins Gehäuse eingepasst ist. Ein Verrutschen des Silikonschlauchs bei der Betätigung des Ventils (Öffnen/Schliessen) oder beim Anschliessen oder Abkoppeln desselben ist daher nicht möglich. Durch die Verwendung von Silikonkunststoff können Ablagerungen, sogenannte Inkrustationen, im Ventil vermieden werden. Zweckmässigerweise sind die Wandstärke des zwischen den Schultern liegenden Leitungsstücks und die Federkraft der Federmittel aufeinander abgestimmt.

Nachfolgend werden Ausführungsbeispiele der Erfindung unter Bezugnahme auf die Figuren beschrieben. Es zeigt:
- Fig.1: eine perspektivische Ansicht des Gehäuses eines erfindungsgemässen Katheterventils mit dem Betätigungshebel in Offenstellung ;
- Fig.2: das Katheterventil von Fig. 1 mit dem Betätigungshebel in Schliessstellung; Fig. 3 eine schematische, perspektivische Ansicht einer Gehäusehälfte mit dem Betätigungshebel in Schliessstellung;
- Fig. 4: eine Seitenansicht eines Katheterventils mit dem Betätigungshebel in Schliessstellung;
- Fig. 5: a) schematisch das Ventil in der Offenstellung;
b) den Betätigungshebel in einer Zwischenposition, in welcher die Leitung voll offen ist;
c) das Ventil in Schliessstellung;
- Fig. 6: schematisch ein Längsschnitt durch das Katheterventil, aus welchem insbesondere die Führungsbahnen für den Betätigungshebel ersichtlich sind;
- Fig. 7: ein Ausführungsbeispiel einer im Katheterventil einsetzbaren Leitung
a) im Längsschnitt;
b) in der Seitenansicht und
c)in perspektivischer Ansicht; und
- Fig. 8: schematisch das Funktionsprinzip einer zweiten Ausführungsform eines Katheterventils in Seitansicht.

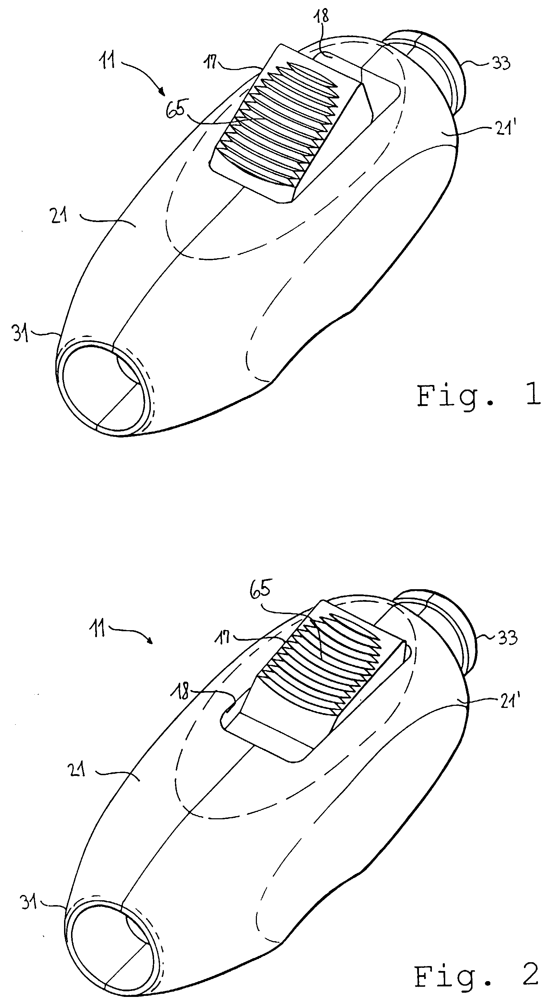

Das Katheterventil 11 gemäss den Figuren 1 bis 7 besteht im wesentlichen aus einem länglichen Gehäuse 13 mit einer das Gehäuse 13 durchsetzenden, elastisch quetschbaren Leitung 15, einem verschwenkbaren Betätigungshebel 17 zum Verschliessen der Leitung 15, und zwei Federn 19, welche mit dem Betätigungshebel 17 zusammenwirken. Mit dem Hebel 17, dessen eine Ende durch eine Öffnung 18 im Gehäuse 13 (Fig. 1 bis 3) von aussen zugänglich ist, kann die Leitung 17 verschlossen und geöffnet werden.

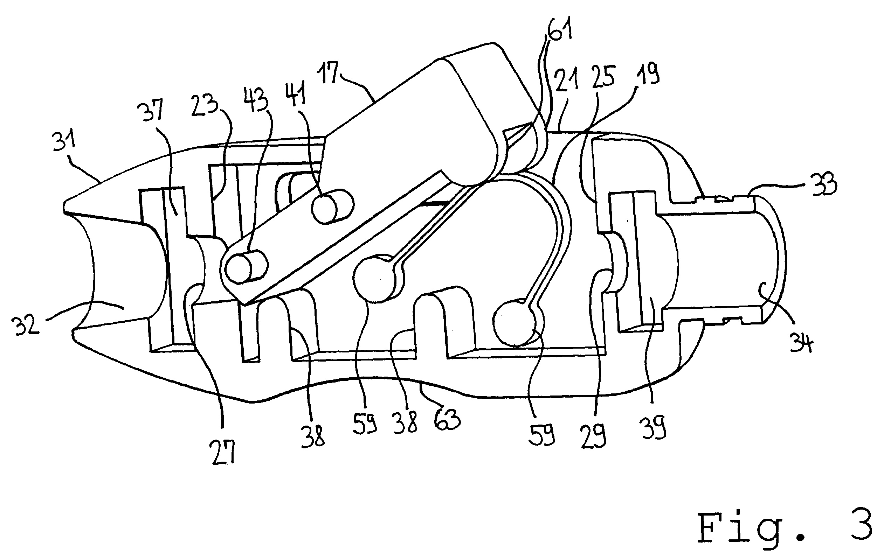

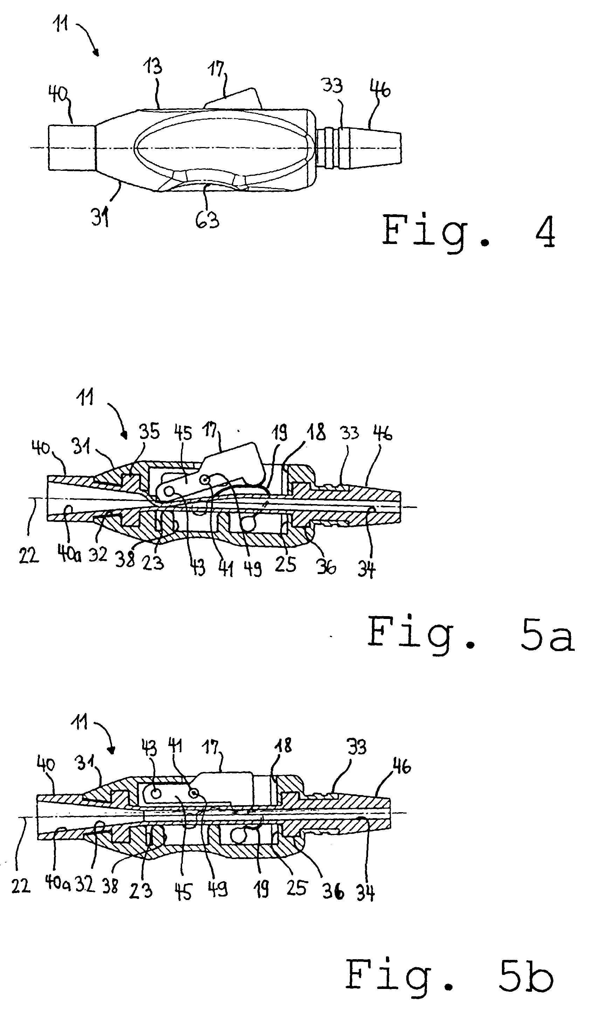

Das Gehäuse 13 des Katheterventils besteht aus zwei Gehäusehälften 21,21' welche bezüglich einer durch die Leitung 15 und den Betätigungshebel 17 gehenden Gehäuselängsmittelebene symmetrisch sind. Die beiden Gehäusehälften 21,21' sind vermittels nicht dargestellter, dem Fachmann geläufiger Mittel zusammensteckbar. Dies ermöglicht, bei einer allfällig auftretenden Inkrustation in der Leitung 15, dieselbe durch eine neue Leitung zu ersetzen. Im mittleren Bereich ist der Gehäusequerschnitt rechteckig mit abgerundeten Kanten. Anschliessend an den mittleren Bereich sind in den Gehäusehälften 21,21' senkrecht zur Gehäuselängsachse 22 verlaufende Querrippen 23,25 mit halbkreisförmigen, fluchtend angeordneten Ausnehmungen 27,29 vorgesehen (Fig. 3). Die Ausnehmungen 27,29 dienen der Aufnahme der Leitung 15 (siehe Figuren 4 bis 7). In Abstand zu den Querrippen 23,25 sind einends ein sich verjüngender Stutzen 31 und andernends ein zylindrischer Stutzen 33 angeformt. Der Stutzen 31 besitzt eine von innen nach aussen sich erweiternde konische Öffnung 32, und der Stutzen 33 einen zylindrischen Durchtritt 34. Im übrigen sind im mittleren Bereich des Gehäuses senkrecht vom Gehäusemantel abstehende Stege 38 vorgesehen, welche als Auflage für die Leitung 15 dienen. Die Enden der Stege 38 sind abgerundet und fluchtend mit den Ausnehmungen 27,29.

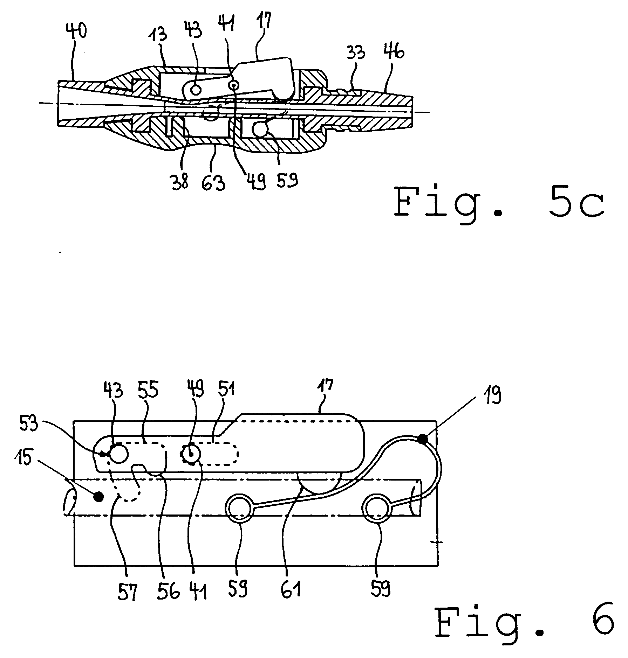

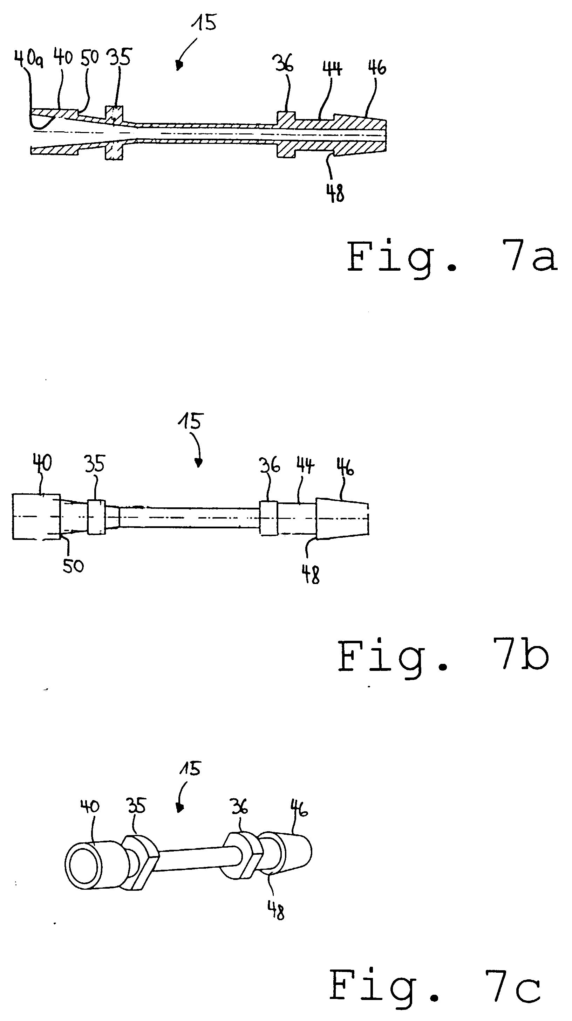

Die Leitung 15 ist bevorzugt ein Silikonschlauch. Der Silikonschlauch durchsetzt das Gehäuse 13 und ragt um ein bestimmtes Mass aus den Stutzen 31,33 heraus (Fig. 5). Der Silikonschlauch besitzt zwei in Abstand voneinander angeordnete Schultern oder Kragen 35,36 (Fig. 7), welche in zwischen den Stutzen 31,33 und den Querrippen 23,25 vorhandene Zwischenräume 37,39 passen (Fig. 3). An den Kragen 35 der Leitung 15 anschliessend ist ein konisches Endteil 40 mit einer konischen Austrittsöffnung 40a vorgesehen. Das Endteil 40 passt formschlüssig in den Innenkonus 32 des Gehäusestutzens 31, wobei dieses mit einem äusseren umlaufenden Rand 50 an der Stirnseite des Stutzens 31 anliegt. Das Endstück 40 lässt den Anschluss beispielsweise eines zu einem Urinauffangbeutel gehörenden Schlauchstücks (nicht gezeigt) zu. Das andere Ende der Leitung 15 besitzt an den Kragen 36 anschliessend einen in den Stutzen 33 passenden zylindrischen Abschnitt 44 und dann einen konischen Anschlussstutzen 46. Der Anschlussstutzen 46 hat einen umlaufenden Rand 48, welcher an der Stirnseite des Stutzens 33 anliegt. An den konischen Anschlussstutzen 46 sind Katheter beliebiger Hersteller anschliessbar. Vermittels der gegenseitig formschlüssigen Gestaltung der Leitungsenden (Ränder 48,50 und Kragen 35,36) und des Gehäuses (Querrippen 27,29 und Stutzen 31,33) sind die Leitung 15 und das Gehäuse 13 relativ zueinander fixiert.

Der Betätigungshebel 17 besitzt zylindrische erste und zweite Zapfen 41,43, welche beidseits von den Seitenkanten 45 des Hebels 17 abstehen. Die ersten Zapfen 41 befinden sich ungefähr in der Mitte des Hebels 17. Sie definieren eine Schwenkachse 49, um welche der Hebel 17 schwenkbar ist (Fig. 5). Die ersten Zapfen 41 sind in den beiden Gehäusehälften 21,21' in je einer zur Leitung 17 parallelen ersten Führungsbahn 51 verschiebbar aufgenommen (Fig. 6).

Die zweiten Zapfen 43 befinden sich am betätigungsfernen Ende des Betätigungshebels 17. Sie sind in den beiden Gehäusehälften 21,21' entlang je einer ungefähr V-förmigen, zweiten Führungsbahn 53 verschiebbar (Fig. 6). Der eine Schenkel 55 der V-förmigen Führungsbahn 53 ist ungefähr fluchtend zur und in seiner Längserstreckung gleich der ersten Führungsbahn 51. Der Schenkel 55 besitzt eine Rastposition, welche durch eine Ausbuchtung 56 der Führungsbahn gebildet ist. Von der Seite gesehen befinden sich der Schenkel 55 und die erste Führungsbahn 51 oberhalb der Leitung 17.

Der zweite Schenkel 57 der V-förmigen Führungsbahn 53 ist gekrümmt und erstreckt sich in einem spitzen Winkel vom ersten Schenkel 55 so weit in Richtung auf die Leitung 17, dass die Leitung 15 verschlossen ist, wenn die zweiten Zapfen 43 in der unteren Endlage sind (Fig. 6c).

Die Federn 19 sind aus einem elastomeren Kunststoff bestehende Streifen, deren Enden in den Geäusehälften 21 schwenkbar angeordnet sind. Die Enden sind als Zylinder 59 ausgebildet, die an ihrer Umfangswand mit der flachen Elastomerfeder verbunden sind. Die Zylinder 59 überragen die Streifen in der Breite um ein bestimmtes Mass. Mit dem überragenden Teil sind die Zylinder 59 in entsprechenden Vertiefungen der Gehäusehälften 21,21' verschwenkbar angeordnet (aus den Figuren nicht ersichtlich). Alternativ können die zylindrischen Enden 59 hohl und auf entsprechende von der Gehäusewand abstehende Stifte aufgesteckt sein. Die zylindrischen Enden 59 der als Federn wirkenden Elastomerstreifen sind in Gehäuselängsrichtung in Abstand voneinander unterhalb des Hebels 17 angeordnet und drücken den einen Arm des Hebels 17 nach oben. Zur Verminderung des Reibungswiderstandes besitzt der Betätigungshebel 17 an der Unterseite zwei halbkreisförmige Erhebungen 61, an welchen die Elastomerfedern anliegen.

Das Katheterventil ist in Grösse und bezüglich Bedienbarkeit ergonomisch ausgebildet. Die Grösse des Gehäuses 13 ist so gewählt, dass dieses gut in der Hand liegt und eine einhändige Bedienung erlaubt. Im weiteren besitzt das Gehäuse am Boden eine Einbuchtung 63, in welcher der Zeigefinger zu liegen kommt, wenn das Ventil mit einer Hand gehalten wird. Zudem besitzt der aus der Öffnung 18 ragende Teil des Hebels 17 eine Vertiefung 65, die einen Eingriff der Daumenfingerbeere erlaubt, sodass der Hebel gut in Längsrichtung verschiebbar ist. Im übrigen sind sämtliche Kanten sowohl des Gehäuses als auch des Betätigungshebels 17 abgerundet, sodass ein Patient sich am Ventil nicht wund scheuern kann.

Der Werkstoff für alle Teile des Katheterventiles 11 ist bevorzugt ein Kunststoff, der eine problemlose Reinigung, Desinfektion und eine Sterilisation des Katheterventiles 11 bei einer Temperatur grösser 130 °C zulässt. Die Sterilisation schafft die Grundlage oder Voraussetzung für eine hygienisch unbedenkliche Verwendung des Katheterventiles.

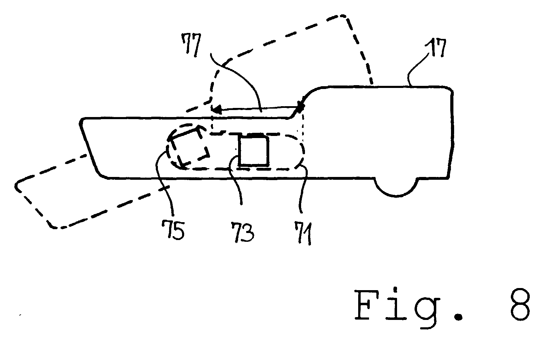

Die modifizierte Ausführungsform gemäss Fig. 8 benötigt lediglich eine Führungsbahn 71. Im Unterschied zum ersten Ausführungsbeispiel besitzt der Betätigungshebel Zapfen 73 mit einem quadratischen Querschnitt. Die Führungsbahn 71 weist an einem Ende eine ungefähr kreisförmige Ausbuchtung 75 auf, deren Durchmesser wenigstens der Diagonale des quadratischen Zapfens 73 entspricht. Der restliche Teil (Pfeil 77) der Führungsbahn 71 besitzt eine Breite, die der Seitenkantenlänge des quadratischen Zapfens entspricht. Befindet sich der Zapfen 73 in der Ausbuchtung 75 (strichliert eingezeichnete Schwenklage des Betätigungshebels), so ist der Betätigungshebel schwenkbar. Befinden sich die Zapfen 73 hingegen im Bereich, welcher durch den Pfeil 77 markiert ist, so ist der Betätigungshebel in der Horizontalen arretiert und das Ventil befindet sich in der Offenstellung.

Das Katheterventil wird wie folgt verwendet (s. Figur 5a bis 5c).: Zuerst wird die Leitung 15 mit einem nicht näher dargestellten Harnkatheter verbunden, indem der Verbindungsschlauch des Harnkathers über den konischen Anschlussstutzen 46 gestülpt und ein ebenfalls nicht näher dargestellter Schlauchstutzen eines Urinauffangbeutels in die konische Austrittsöffnung 40a der Leitung 15 gesteckt wird.

In der Normalstellung ist das Ventil 11 geschlossen (Fig. 5a). Die Federn 19 üben eine Vorspannung auf den Betätigungshebel 17 aus, welche ausreicht, um den Silikonschlauch 15 so weit zusammenzudrücken, dass ein Flüssigkeitsdurchtritt unmöglich ist. Dabei wirkt das abgerundete Ende des Betätigungshebels 17 mit dem oben ebenfalls abgerundeten Steg 38 zusammen. In der Schliessstellung sind die Federn 19 U-förmig vorgespannt.

Zum Öffnen des Ventils drückt der Patient den Betätigungshebel 17 gegen die Kraft der Federn 19 nach unten, wodurch der volle Leitungsquerschnitt freigegeben ist (Fig. 5b). Da die Federn 19 dem durch den Patienten ausgeübten Druck nachgeben können, indem die Federschenkel auf die Seite schwenken, ist die für das Öffnen des Ventils nötige Kraft geringer als bei Federn mit linearer Federcharakteristik. Anschliessend wird der Betätigungshebel 17 in die Arretierstellung gebracht, indem dieser so weit nach rechts verschoben wird, bis die Zapfen 43 in der Ausbuchtung 56 einrasten können (Fig. 6a). In dieser Offenstellung der Leitung 15 wird der Betätigungshebel 17 wiederum durch die Federn 19 unter Vorspannung gehalten, sodass das Ventil durch eine in Längsrichtung wirkende Kraft nicht versehentlich geöffnet werden kann.

Die vorliegende Erfindung beschränkt sich nicht auf die gezeigten Ausführungsbeispiele. Es sind verschiedene Modifikationen denkbar. Zum Beispiel kann das Gehäuse so gestaltet werden, dass der Betätigungshebel auch in der Schliessstellung innerhalb der Konturen des Gehäuses liegt, sodass das Ventil auch nicht versehentlich geöffnet werden kann, wenn ein Patient in der Nacht auf dem Ventil liegt. Auch können die Federn so angeordnet sein, dass diese das Ventil offen halten, sodass das Ventil gegen die Federkraft der Federn geschlossen wird. In diesem Fall ist die Rastposition so angeordnet, dass der Betätigungshebel in der Schliessstellung arretiert ist (Fig. 5: Schenkel 55 mit nach oben orientierter Ausbuchtung würde am entgegengesetzten Ende des Schenkels 57 vorgesehen). Das vorgeschlagene Katheterventil kann als Harnkatheterventil und auch zum Verschliessen einer Infusionsleitung für die paraenterale Ernährung verwendet werden.

## Patentansprüche

1. Katheterventil (11) mit
- einem Gehäuse (13);
- einer elastisch verformbaren Leitung (15) , welche sich durch das Gehäuse (13) erstreckt;
- Mitteln, um die Leitung (15) zu verschliessen und zu öffnen; und
- einer ersten und einer zweiten Anschlussstelle zum Verbinden der Leitung (15) beispielsweise mit einem Katheter und einem Auffangbehälter; und
**gekennzeichnet durch**
- Schliessmittel mit wenigstens einem im Gehäuse in wenigstens einer bestimmten Lage verschwenkbaren Betätigungshebel (17), welcher wenigstens eine erste und eine zweite Stellung einnehmen kann, wobei die Leitung (15) in der ersten Stellung offen und in der zweiten Stellung verschlossen ist; und
- Federmittel (19), welche mit dem Betätigungshebel (17) in Verbindung stehen und diesen wenigstens in der ersten oder zweiten Stellung zu halten vermögen; und
- Mittel (49,51,43,55;73,77), um den Betätigungshebel (17) in wenigstens der zweiten oder der ersten Stellung festzustellen.

2. Katheterventil nach Anspruch 1, dadurch gekennzeichnet, dass die Federmittel (19) so angeordnet sind, dass die auf den Betätigungshebel (17) wirkende Federkraft die Leitung (15) verschlossen hält, und die Feststellmittel den Hebel (17) in der ersten Stellung, d.h. in der Offenstellung, arretieren.

3. Katheterventil nach Anspruch 1 oder 2, dadurch gekennzeichnet, dass die Feststellmittel in Gestalt wenigstens einer Rastposition (55;77), in welche der Betätigungshebel (17) verschiebbar ist, realisiert sind.

4. Katheterventil nach Anspruch 3, dadurch gekennzeichnet, dass die Rastposition durch eine in einem Winkel zur kreisförmigen Schwenkbewegung des Betätigungshebels (17) angeordnete erste Führungsbahn (51) gebildet ist.

5. Katheterventil nach einem der Ansprüche 1 bis 4, dadurch gekennzeichnet, dass der Betätigungshebel (17) wenigsten einen ersten Zapfen (41) aufweist, welcher in der ersten Führungsbahn (51) schwenk- und in Leitungslängsrichtung verschiebbar geführt ist.

6. Katheterventil nach einem der Ansprüche 1 bis 5, dadurch gekennzeichnet, dass der Betätigungshebel (17) wenigstens einen zweiten Zapfen (43) aufweist, welcher im Gehäuse in einer zweiten wenigstens teilweise kurven- oder kreisförmigen Führungsbahn (53) geführt ist, welche so orientiert ist, dass der Hebel (17) die Leitung (15) in der einen Stellung verschliesst und in der anderen offen hält.

7. Katheterventil nach Anspruch 6, dadurch gekennzeichnet,
- dass die zweite Führungsbahn (53) ungefähr V-förmig ist,
- dass der eine Schenkel (55) der ungefähr V-förmigen Führungsbahn (53) ungefähr parallel zur Gehäuselängsachse (22) angeordnet ist, sodass der Betätigungshebel (17) in Längsrichtung verschiebbar ist, und
- dass der besagte Schenkel (55) eine quer zu seiner Längsachse sich erstreckende, als Rastposition dienende Ausbuchtung (56) zur Aufnahme des zweiten Zapfens (43) besitzt.

8. Katheterventil nach Anspruch 6 oder 7, dadurch gekennzeichnet, dass die zweiten Zapfen (43) des Betätigungshebels (17) durch die Federkraft der Federmittel (19) in der Ausbuchtung (56) gehalten sind.

9. Katheterventil nach einem der Ansprüche 1 bis 8, dadurch gekennzeichnet, dass die Federmittel (19) durch wenigstens eine Blattfeder, z.B. eine Feder aus einem elastomeren Kunststoff, gebildet sind.

10. Katheterventil nach einem der Ansprüche 1 bis 9, dadurch gekennzeichnet, dass die Federmittel aus beidseitig der Leitung angeordneten Federelementen (19) bestehen.

11. Katheterventil nach Anspruch 10, dadurch gekennzeichnet, dass das Federelement (19) ein länglicher Kunststoffstreifen ist, dass die Enden des Kunststoffstreifens je eine quer zur Längsrichtung des Streifens sich erstreckende zylindrische Verdickung (59) aufweisen, welche im Gehäuse (13) schwenkbar angeordnet sind.

12. Katheterventil nach einem der Ansprüche 1 bis 11, dadurch gekennzeichnet, dass das Gehäuse (13) aus zwei länglichen Halbschalen (21,21') besteht und eine Öffnung (18) besitzt, durch welche sich das eine Ende des Hebels (17) erstreckt.

13. Katheterventil nach Anspruch 12, dadurch gekennzeichnet, dass das durch die Öffnung (18) zugängliche Ende des Betätigungshebels (17) eine Vertiefung (65) aufweist.

14. Katheterventil nach einem der Ansprüche 1 bis 13, dadurch gekennzeichnet, dass die Anschlussstellen in einer konischen Austrittsöffnung (40a) resp. einem konischen Anschlussstutzen (46) der Leitung (15) bestehen.

15. Katheterventil nach Anspruch 14, dadurch gekennzeichnet, dass die Leitung (15) zwei in Abstand voneinander angeordnete Schultern (35,36) aufweist, welche in entsprechenden Zwischenräumen (37,39) der Gehäusehälften (21,21') aufgenommen sind, sodass die Leitung (15) und das Gehäuse (13) relativ zueinander fixiert sind.

16. Katheterventil nach einem der Ansprüche 1 bis 15, dadurch gekennzeichnet, das Ventil (11) vollständig aus Kunststoff und die Leitung (15) aus Silikonkunststoff hergestellt ist.
